(19) Europäisches Patentamt / European Patent Office / Office européen des brevets

(11) **EP 4 714 490 A2**

(12) **EUROPEAN PATENT APPLICATION**

(43) Date of publication:
25.03.2026   Bulletin 2026/13

(21) Application number: 25219282.8

(22) Date of filing: 05.01.2021

(51) International Patent Classification (IPC):
**A61N 1/08** (2006.01)

(52) Cooperative Patent Classification (CPC):
**A61N 1/3925;** A61N 2001/083

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority:   05.01.2020   US 202062957245 P

(62) Document number(s) of the earlier application(s) in accordance with Art. 76 EPC:
**21701166.7 / 4 084 864**

(71) Applicant: **Impulse Dynamics NV**
**Willemstad (CW)**

(72) Inventor: **PRUTCHI, David**
**Voorhees, New Jersey 08043 (US)**

(74) Representative: **Cohausz & Florack**
**Patent- & Rechtsanwälte**
**Partnerschaftsgesellschaft mbB**
**Bleichstraße 14**
**40211 Düsseldorf (DE)**

Remarks:
•This application was filed on 28.11.2025 as a divisional application to the application mentioned under INID code 62.
•Claims filed after the date of filing of the application / after the date of receipt of the divisional application (Rule 68(4) EPC).

(54) **LEAD CONDITION TESTING IN AN IMPLANTED CARDIAC DEVICE**

(57)     Some embodiments relate to a method of testing a lead condition in an implanted cardiac device comprising a first defibrillation lead and a second non-defibrillation lead, the method comprising: measuring impedance between the first defibrillation lead and the second non-defibrillation lead by applying a test pulse; and determining a condition of at least one of the defibrillation lead and the non-defibrillation lead according to the measured impedance value.

FIG. 1B

**Description**

RELATED APPLICATIONS

This application claims the benefit of priority under 35 USC §119(e) of U.S. Provisional Patent Application No. 62/957,245 filed January 5, 2020; the contents of which are incorporated herein by reference in their entirety.

FIELD AND BACKGROUND OF THE INVENTION

The present invention, in some embodiments thereof, relates to assessment of lead condition and/or lead integrity in an implanted device, and, more particularly, but not exclusively, to impedance measurements in an implanted device comprising a defibrillation lead and one or more non-defibrillation leads.

A paper titled *"Advanced ICD Troubleshooting: Part II"* by Charles D. Swerdlow; Paul A. Friedman (Pacing Clin Electrophysiol. 2006;29(1):70-96.) discloses: *"High-Voltage Impedance. Shocking-pathway impedance normally is 25-75 Ω in transvenous systems. Values outside this range suggest a conductor defect (high impedance) or insulation failure/short circuit (low impedance). Periodic assessment of the impedance of the high-voltage electrodes using weak test pulses may detect a loss of lead integrity before inappropriate or ineffective therapies occur. In Medtronic and Guidant ICDs, painless pulses are delivered automatically at periodic intervals to create trend plots. In St. Jude ICDs, high-voltage lead impedance is assessed by delivering a 12-V test pulse using the programmer command. Patients feel this pulse and may perceive it to be uncomfortable. High-voltage impedance measured by weak pulses correlates well with values measured by high-energy shocks. [43, 44J In Guidant ICDs, impedance values recorded painlessly are comparable to those recorded during high-voltage shock. In older Medtronic ICDs, the pulse is delivered between the lead tip and the distal coil, resulting in nominal low-voltage impedance values of 11-20 Ω, significantly lower than the impedance of a high-energy shock. If a proximal coil is present, its integrity is not assessed. Medtronic Marquis™ ICDs and newer models report independent proximal and distal coil impedances that closely approximate those of high-voltage shocks. When a lead defect is suspected, a full-output shock may be necessary to evaluate lead integrity. Partial insulation defects may not be identified by low-energy pulses that deliver insufficient current to activate the shorted high-output protection feature (see below)....* " (Pages 8-9)*

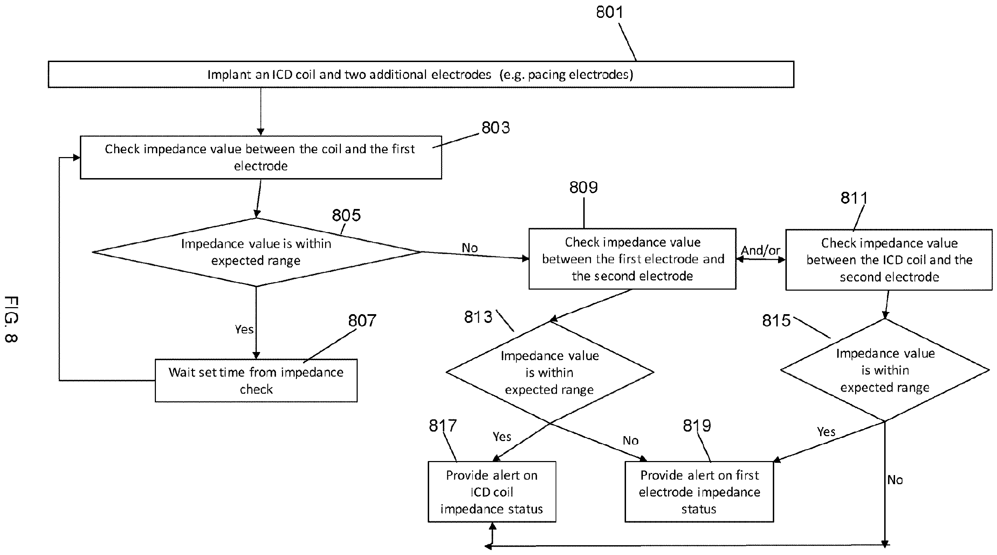

*"Protecting ICDs from High-Voltage, Short Circuits. Low impedance on the shock electrodes indicates an insulation failure that diverts shock current from the heart. It may produce sufficient peak current that the ICD's output circuit fails by "electrical overstress. "[58] For example, an insulation failure in the pocket may produce a short circuit from the high-voltage coil to the can with a resistance of 8 Ω. A maximum-output (~800 V) shock would result in a peak current of 100 A in the high-voltage output circuit, causing it to fail catastrophically. [59] Shorting between two active intracardiac electrodes of opposite polarity can cause the same effect. To prevent electrical-overstress component failure, modern ICDs protect the output circuit by aborting the shock pulse immediately if the current in the defibrillation pathway is excessive. Depending on the manufacturer, this corresponds to a series lead impedance of 15-20 Ω. The presumption is that the short circuit diverts current from the heart, preventing shocks from terminating VT/VF. In addition to preserving the generator, this feature has a safety benefit. Simultaneous insulation failure of the rate-sensing and high-voltage components of RV leads may present as oversensing. Diverting the resultant inappropriate shock prevents unnecessary pain. Further, it may prevent fatal proarrhythmiafrom a weak shock delivered in the vulnerable period with no effective means to rescue the patient [60] (see Fig. 8). "* (Page 14)

SUMMARY OF THE INVENTION

According to an aspect of some embodiments of the invention there is provided a method of testing a lead condition in an implanted cardiac device comprising a first defibrillation lead and a second non-defibrillation lead, the method comprising: measuring impedance between the first defibrillation lead and the second non-defibrillation lead by applying a test pulse; and determining a condition of at least one of the defibrillation lead and the non-defibrillation lead according to the measured impedance value.

In some embodiments, the second non-defibrillation lead is configured for applying cardiac contractility modulation stimulation.

In some embodiments, applying a test pulse is during applying of the cardiac contractility modulation stimulation.

In some embodiments, applying a test pulse is during a ventricle refractory period, before or after applying of the cardiac contractility modulation stimulation.

In some embodiments, the defibrillation lead comprises a coil, and the non-defibrillation lead comprises a ring electrode and a tip electrode; and measuring impedance comprises measuring impedance between at least one of the coil and the ring electrode; the coil and the tip electrode.

In some embodiments, determining a condition of at least one of the defibrillation lead and the non-defibrillation lead is by one or more of: comparing the impedance value to one or more of: a look-up table; one or more previously measured values; one or more predefined values.

In some embodiments, the method comprises repeating the measuring once a day.

In some embodiments, the method comprises issuing an alert regarding the lead condition.

In some embodiments, determining a lead condition comprises determining at least one of: lead fracture, dislodgment of the lead, lead insulation, lead connectivity, lead deformation.

In some embodiments, at least a portion of the first lead and at least a portion of the second lead is implanted in contact with a wall of the right ventricle of the heart.

In some embodiments, the defibrillation lead comprises a coil, ring electrode and tip electrode; and the non-defibrillation electrode comprises a ring electrode and a tip electrode; and measuring impedance comprises measuring inter-lead impedance between one of the coil, ring electrode and tip electrode of the defibrillation lead and one of the ring electrode and tip electrode of the non-defibrillation lead.

In some embodiments, the non-defibrillation lead comprises a cardiac contractility modulation lead or a pacing lead.

According to an aspect of some embodiments of the invention there is provided an implantable cardiac device comprising: a first defibrillation lead including a coil, a ring electrode and a tip electrode; a second non-defibrillation lead including a ring electrode and a tip electrode; and circuitry for controlling and activating the coil and electrodes, the circuitry including at least one grounded resistor electrically connected to the second non-defibrillation lead; the circuitry configured to measure current across the grounded resistor in response to an applied test pulse to obtain an indication of a condition of at least the first defibrillation lead or a portion thereof.

In some embodiments, the circuitry is at least partially disposed within a housing of the cardiac device, the leads extending from within the housing.

In some embodiments, the current measured across the grounded resistor in response to an applied test pulse is used for calculating impedance between the coil and the device housing.

In some embodiments, the circuitry is configured to calculate the impedance between the coil and device housing using a pre-determined linearizing factor.

In some embodiments, the circuitry is programmed for timing the test pulse during an expected total refractory period of the cardiac cycle.

In some embodiments, the non-defibrillation lead is a cardiac contractility modulation lead, and the circuitry is programmed for timing the measurement of current on the grounded resistor during applying of a cardiac contractility modulation signal.

According to an aspect of some embodiments of the invention there is provided a method for assessing a lead condition an implantable cardiac device including a defibrillation lead having a coil and at least one non-defibrillation lead having at least one electrode, the method comprising: applying (also referred to as "first applying") a test pulse to measure a baseline impedance between the defibrillation coil and the device housing; applying (also referred to as "second applying"), a test pulse to measure a baseline impedance between the electrode of the non-defibrillation lead and the defibrillation coil; applying (also referred to as "third applying"), periodically, a test pulse to measure impedance between the electrode of the non-defibrillation lead and the defibrillation coil; and estimating, according to a difference between a currently measured impedance level measured at the third applying and one or both of the baseline measurements, a current impedance between the coil and the device housing to assess a condition of the defibrillation lead.

In some embodiments, the third applying is performed more frequently than the first and second applying.

In some embodiments, applying periodically comprises applying every 24 hours.

According to an aspect of some embodiments of the invention there is provided a method of testing lead integrity in an implantable cardiac device comprising at least one lead including at least two electrodes, comprising: during the total ventricle refractory period of the cardiac cycle, applying a test pulse having a selected duration and voltage; measuring impedance between the at least two electrodes and/or between an electrode and the device housing; and determining a condition of the lead according to the measured impedance value.

In some embodiments, one of the at least two electrodes comprises a defibrillation coil.

In some embodiments, one of the at least two electrodes comprises a ring electrode or a tip electrode.

According to an aspect of some embodiments of the invention there is provided a method for assessing a lead condition in an implanted cardiac device comprising a defibrillation lead and at least one non-defibrillation lead, comprising: measuring impedance between the defibrillation lead and the non-defibrillation lead by conducting current via a first electrical path of the device circuitry; checking if the patient has sensed or was in pain during the measuring; and measuring impedance between the defibrillation lead and the non-defibrillation lead by conducting current via a second electrical path of the device circuitry, if the patient has sensed or felt pain during the initial measuring.

According to an aspect of some embodiments of the invention there is provided a method for assessing a lead condition in an implanted cardiac device comprising a defibrillation coil and at least two electrodes, comprising: measuring impedance between the coil and a first electrode; measuring impedance between the coil and a second electrode or between the first and second electrodes; and assessing integrity of the coil, first and second electrodes in accordance with the measured impedance values.

In some embodiments, the first and second electrodes are implanted within or in contact with tissue of the heart.

According to an aspect of some embodiments there is provided an implantable cardiac device comprising:

a first defibrillation lead;
a second non-defibrillation lead;
circuitry for controlling the leads and for measuring impedance, the circuitry configured to apply a test pulse to measure impedance between the first defibrillation lead and the second non-defibrillation lead, and to determine a condition of at least one of the defibrillation lead and the non-defibrillation lead according to the measured impedance value.

In some embodiments, the second non-defibrillation lead is configured for applying cardiac contractility modulation stimulation.

In some embodiments, the circuitry is configured to time the test pulse during applying of the cardiac contractility modulation stimulation via the second non-defibrillation lead.

In some embodiments, the circuitry is configured to time the test pulse during a ventricle refractory period.

In some embodiments, the defibrillation lead comprises a coil, and the non-defibrillation lead comprises a ring electrode and a tip electrode; and wherein the impedance is measured between at least one of: the coil and the ring electrode; the coil and the tip electrode.

In some embodiments, the circuitry is configured to determine the condition of at least one of the defibrillation lead and the non-defibrillation lead by one or more of: comparing the measured impedance to one or more of: a look-up table; one or more previously measured impedance values; one or more predefined impedance values.

In some embodiments, the circuitry is configured to time the test pulse to perform measurement once every 24 hours.

In some embodiments, the circuitry is configured to generate and issue an alert regarding the determined lead condition.

In some embodiments, the determined lead condition comprises at least one of: lead fracture, dislodgment of the lead, lead insulation, lead connectivity, lead deformation.

In some embodiments, the defibrillation lead comprises a coil, ring electrode and tip electrode; and the non-defibrillation lead comprises a ring electrode and a tip electrode; and the impedance is measured between one of the coil, ring electrode and tip electrode of the defibrillation lead and one of the ring electrode and tip electrode of the non-defibrillation lead.

In some embodiments, the non-defibrillation lead comprises a cardiac contractility modulation (cardiac contractility modulation) lead or a pacing lead.

According to an aspect of some embodiments there is provided an implantable cardiac device comprising:

a housing;
a defibrillation lead having a coil, the defibrillation lead extending from the housing;
a non-defibrillation lead having at least one electrode, the non-defibrillation lead extending from the housing;
circuitry for controlling and activating the defibrillation lead and the non-defibrillation lead and for measuring impedance, the circuitry configured to:

apply a test pulse to measure a baseline impedance between the coil of the defibrillation lead and the housing;
apply a test pulse to measure a baseline impedance between the at least one electrode of the non-defibrillation lead and the coil of the defibrillation lead;
apply, periodically, a test pulse to measure impedance between the at least one electrode of the non-defibrillation lead and the coil of the defibrillation lead; and
estimate, according to a difference between a currently measured impedance level measured between the at least one electrode of the non-defibrillation lead and the coil of the defibrillation lead and one or both of the baseline impedance measurements, a current impedance between the coil of the defibrillation lead and the housing; and
assess a condition of the defibrillation lead based on the estimated current impedance.

In some embodiments, the circuitry is configured to apply the test pulse to measure impedance between the at least one electrode of the non-defibrillation lead and the coil of the defibrillation lead at least every 24 hours.

Unless otherwise defined, all technical and/or scientific terms used herein have the same meaning as commonly understood by one of ordinary skill in the art to which the invention pertains. Although methods and materials similar or equivalent to those described herein can be used in the practice or testing of embodiments of the invention, exemplary methods and/or materials are described below. In case of conflict, the patent specification, including definitions, will control. In addition, the materials, methods, and examples are illustrative only and are not intended to be necessarily limiting.

Implementation of the method and/or system of embodiments of the invention can involve performing or completing selected tasks manually, automatically, or a combination thereof. Moreover, according to actual instrumentation and equipment of embodiments of the method and/or system of the invention, several selected tasks could be implemented by hardware, by software or by firmware or by a combination thereof using an operating system.

For example, hardware for performing selected tasks according to embodiments of the invention could be implemented as a chip or a circuit. As software, selected tasks according to embodiments of the invention could be implemented as a plurality of software instructions being executed by a computer using any suitable operating system. In an exemplary embodiment of the invention, one or more tasks according to exemplary embodiments of method and/or system as described herein are performed by a data processor, such as a computing platform for executing a plurality of instructions. Optionally, the data processor includes a volatile memory for storing instructions and/or data and/or a non-volatile storage, for example, a magnetic hard disk and/or removable media, for storing instructions and/or data. Optionally, a network connection is provided as well. A display and/or a user input device such as a keyboard or mouse are optionally provided as well.

BRIEF DESCRIPTION OF THE SEVERAL VIEWS OF THE DRAWINGS

Some embodiments of the invention are herein described, by way of example only, with reference to the accompanying drawings. With specific reference now to the drawings in detail, it is stressed that the particulars shown are by way of example and for purposes of illustrative discussion of embodiments of the invention. In this regard, the description taken with the drawings makes apparent to those skilled in the art how embodiments of the invention may be practiced.

In the drawings:

FIG. 1A is schematic illustration of an implantable device comprising a defibrillation lead and at least one cardiac contractility modulation lead, according to some embodiments;

FIG. 1B schematically illustrates cross-measurements of impedance between components of two leads of an implantable device, according to some embodiments;

FIG. 1C is a schematic diagram of impedance measurements in a cardiac device comprising two leads, according to some embodiments;

FIGs. 2A-D are flowcharts of general methods for assessing a lead condition by measuring impedance in a cardiac device, according to some embodiments;

FIG. 3 is a schematic diagram of circuitry apparently used in implantable pulse generator devices (IPG) for impedance measurement;

FIG. 4 is a schematic diagram of circuitry suitable for impedance measurement in a device comprising a defibrillation lead and at least one cardiac contractility modulation lead, according to some embodiments;

FIG. 5 is a schematic diagram of circuitry suitable for impedance measurement in a device comprising a defibrillation lead and at least one pacing lead, according to some embodiments;

FIG. 6 is a schematic diagram showing components of an implantable ICD/cardiac contractility modulation device, according to some embodiments;

FIG. 7 is an example of an impedance look up table for assessing a lead condition, according to some embodiments;

FIG. 8 is a flowchart of a method for estimating a connectivity condition in a device comprising a defibrillation coil and two electrodes, according to some embodiments; and

FIG. 9 is a graphical representation of a cardiac cycle showing the relative timing of applying of a cardiac contractility modulation stimulation and of a test pulse for impedance measurement, according to some embodiments.

DESCRIPTION OF SPECIFIC EMBODIMENTS OF THE INVENTION

The present invention, in some embodiments thereof, relates to assessment of lead integrity in an implanted device, and, more particularly, but not exclusively, to impedance measurements in an implanted cardiac device comprising a defibrillation lead and one or more non-defibrillation leads. In some embodiments, the device comprises a defibrillation coil and one or more electrodes.

A broad aspect of some embodiments relates to assessing integrity of at least a part of the circuitry of an implanted cardiac device while substantially avoiding or reducing pain and/or sensation to the patient. In some embodiments, a condition of one of one or more leads of the device, which are positioned at least in part inside the heart, is assessed by measuring impedance, for example impedance between components of a same single lead and/or impedance between components of two different leads.

An aspect of some embodiments relates to reducing or avoiding sensation and/or pain to the patient during impedance measurements by avoiding a measurement path, which significantly stimulates nerves. Optionally, direct and/or indirect stimulation of the nerves is avoided or reduced. In some embodiments, measurement paths and/or signal amplitudes are selected to reduce or prevent stimulation of nerves. Reducing or avoiding stimulation of nerves may include reducing or avoiding stimulation of sympathetic nerves and/or parasympathetic nerves.

In some embodiments, for example in a device including two more leads, various measurement paths are available (e.g. various cross-measurement paths between components of the two different leads). Optionally, if measurement via a certain path results in pain or sensation to the patient, an alternative path may be selected.

An aspect of some embodiments relates to assessing a condition of at least one implanted lead by measuring impedance between a defibrillation lead and a non-defibrillation lead. In some embodiments, the non-defibrillation lead comprises a pacing lead. In some embodiments, the non-defibrillation lead comprises a cardiac contractility modulation lead. In some embodiments, the non-defibrillation lead comprises a sensing lead.

In some embodiments, at least a portion of each of the leads is implanted inside the heart, such as inside the right ventricle. Optionally, the lead is implanted within the muscle tissue of the heart. Optionally, each of the leads extends from within the heart to an external device, which generates the applied signals, and is optionally implanted in the subclavian area.

In some embodiments, each lead comprises one or more of: a coil, a ring electrode, and a tip electrode. In some embodiments, cross impedance measurements are performed between pairs of components, whereby a first component of the pair is configured on the defibrillation lead and a second component of the pair is configured on the non- defibrillation lead.

In some embodiments, the cardiac device comprises more than two leads, for example a defibrillation lead and two non-defibrillation leads (e.g. cardiac contractility modulation leads, pacing leads), and various combinations of impedance measurements are performed, e.g. between the first and second lead, between the first and third lead, between the second and third lead. Optionally, consecutive cross measurements are performed if an initially selected measurement path induces pain or is otherwise sensed by the patient. Optionally, consecutive cross measurements are performed so that one or more measurement results are used as a reference value for another measurement. Optionally, consecutive measurements are performed to assess in which out of, for example, three components (e.g. a coil and two electrodes) a defect or a connectivity problem exists.

A potential advantage of cross-measurements such as cross measurements performed between the at least two leads may include avoiding the need to conduct current between the defibrillation lead (for example the coil of the lead) and the device itself (coil-to-can (canister) configuration) for measuring impedance. As the coil-to-can path passes through sensory nerves, avoiding such measurement may contribute to reducing pain or sensation to the patient, at least during impedance measurements.

In some embodiments, impedance is measured by applying a test pulse. Based on the resulting impedance value, a condition of the lead may be assessed. Some examples of lead conditions which may be detected according to the measured impedance value include lead fracture, dislodgment of the lead, an insulation defect, connectivity defects, a cut-off in a lead wire and/or other conditions related to lead integrity, conduction capabilities, deformation, changes in lead material properties and the like.

In some examples, assessment of the lead condition via impedance measurements is performed periodically and/or at pre-set timing and/or at pre-set intervals, for example every 5 minutes, every 50 minutes, every 30 minutes, every hour, every 4 hours, every 12 hours, every 24 hours, every 2 days, every week, or intermediate, longer or shorter time intervals.

An aspect of some embodiments relates to an implantable ICD/cardiac contractility modulation (device comprising a defibrillation lead, a cardiac contractility modulation lead, and circuitry configured for applying a test pulse for measuring impedance between the two leads during applying of the cardiac contractility modulation signal. In some embodiments, due to the cardiac contractility modulation lead's suitability to apply a high voltage signal, the test pulse applied for impedance measurement can be applied at a relatively high voltage, for example between, 8-16 V, 5-10 V, 6-12 V, 4-8 V or intermediate, higher or lower values. In some embodiments, a duration of the applied test pulse is as short as 6 $\mu$sec, 10 $\mu$sec, 8 $\mu$sec, 5 $\mu$sec or intermediate, longer or shorter time periods. A potential advantage of a short test pulse may include reduced pain or even no sensation to the patient.

An aspect of some embodiments relates to applying the test pulse for measuring impedance during the ventricle refractory period. Optionally, the test pulse is applied during the absolute refractory period. A potential advantage of applying the test pulse during the ventricle refractory period may include improving safety of the implanted device because a new action potential cannot be elicited, therefore the applied pulse is not likely to stimulate undesired contraction.

In some embodiments, the test pulse for measuring impedance is applied during the ventricle refractory period, but not during applying of a cardiac contractility modulation signal.

In some embodiments, the test pulse does not cause a therapeutic effect. In some embodiments, parameters of the test pulse (such as voltage, duration, timing of applying of the test pulse) are selected so as not to stimulate cardiac contraction. In some embodiments, the test pulse does not affect cardiac function. In some embodiments, test pulse parameters are selected so as not to interfere with therapeutic signals applied by the implanted cardiac device, such as pacing or cardiac contractility modulation signals. In some embodiments, the test pulse is applied solely for the purpose of assessing lead condition (e.g. lead integrity).

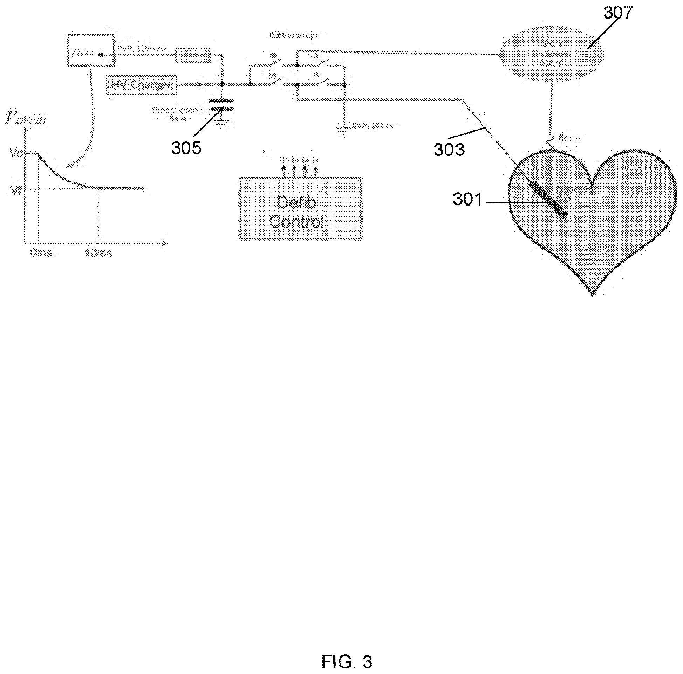

An aspect of some embodiments relates to device circuitry in which impedance is measured by measuring current across a grounded resistor. Optionally, the non-defibrillation lead is grounded via that same resistor; therefore, no additional circuitry is required for impedance measurement. A potential advantage of using existing device circuitry for measuring impedance may include maintaining the device volume at a minimum, due to that no additional components are required for the measurement. Another potential advantage of measuring impedance by measuring current across a resistor, as opposed to, for example, measuring discharge of a defibrillation capacitor (see FIG. 3) may include the ability to detect impedance even when applying a test pulse of relatively low voltage. When measuring impedance via the capacitor, the applied test pulse voltage needs to be high enough to enable detection of a small change in the capacitor discharge. Therefore, measuring current across the resistor, in accordance with some embodiments, may provide for a higher sensitivity of the measurement.

An aspect of some embodiments relates to estimating coil-to-can impedance (between a coil of a defibrillation electrode and a housing of the cardiac device) without directly performing the measurement between the two components. In some embodiments, a method for estimating coil-to-can impedance involves applying a test pulse to measure a baseline coil-to-can impedance (optionally once or a limited number of times); then measure a baseline ring electrode-to-coil impedance, and then repeat the ring electrode-to-coil measurement, optionally periodically. In some embodiments, a current coil-to-can impedance is estimated or calculated according to a difference between a currently measured ring electrode-to-coil impedance and the baseline ring electrode-to-coil impedance, and taking into account the baseline coil-to-can impedance. A potential advantage of this method may include avoiding the need for multiple periodic measurements of coil-to-can impedance, thereby reducing the number of times current is conducted along a path along which sensory nerves are located, potentially reducing pain to the patient.

As referred to herein, assessment or estimation of lead integrity may refer to assessing a condition of: a defibrillation lead or portions thereof, such as the defibrillation coil; a non-defibrillation lead (e.g. a pacing lead, a cardiac contractility modulation lead) or portions thereof; and/or other circuitry or electrical connections of the cardiac device.

As referred to herein, a non-defibrillation lead may refer to: a lead which is not suitable for passing current above a certain threshold, such as current required for defibrillation; a lead which does not include a defibrillation coil or other defibrillation element; a lead suitable for applying pacing signals and/or for applying cardiac contractility modulation signals, a lead comprising one or more electrodes for applying and/or sensing electrical signals (in an example, electrocardiogram).

For purposes of better understanding some embodiments of the present invention, reference is first made to FIG. 3, showing circuitry useable in implantable pulse generator devices (IPG) for impedance measurement.

In the diagram, an example of an ICD (implantable cardioverter defibrillator) device including a defibrillation coil 301 is shown. For assessing the integrity of lead 303 on which the coil is configured, impedance of the coil 301 may be measured by delivering a pulse, from the defibrillation capacitor 305, between coil 301 and the device enclosure 307 ("can"). To generate a measureable change in capacitance, the pulse delivered is required to be of a high enough amplitude and of sufficient width, for example, at least 12V delivered over a time period of between 5-10 msec. These requirements are dictated by the capacitance of capacitor 305 (which, in some examples, is 150μF) and by the properties and construction of the switching circuit (indicated by switches S1-S4).

In some cases, applying of a pulse having the above-mentioned properties may cause pain and/or otherwise be strongly sensed by the patient. Therefore, in some cases, impedance measurements are performed in a clinical setting, and require monitoring of a clinician. This may prevent from performing routine, periodic measurements at short time intervals (e.g. daily or even hourly), which may contribute to identifying a defect in time, reducing risk and potential damage to the device and/or the patient.

In some cases, additional dedicated circuitry is added to the device for the purpose of impedance measurement. This may take up existing device volume or enlarge it. The added circuitry often requires additional isolation, which may increase the device weight and/or dimensions.

In some pacemaker devices, impedance is measured via discharge of the capacitor throughout the duration of the pacing pulse.

Before explaining at least one embodiment of the invention in detail, it is to be understood that the invention is not necessarily limited in its application to the details of construction and the arrangement of the components and/or methods set forth in the following description and/or illustrated in the drawings and/or the Examples. The invention is capable of other embodiments or of being practiced or carried out in various ways.

Before explaining at least one embodiment of the invention in detail, it is to be understood that the invention is not necessarily limited in its application to the details set forth in the following description or exemplified by the Examples. The invention is capable of other embodiments or of being practiced or carried out in various ways.

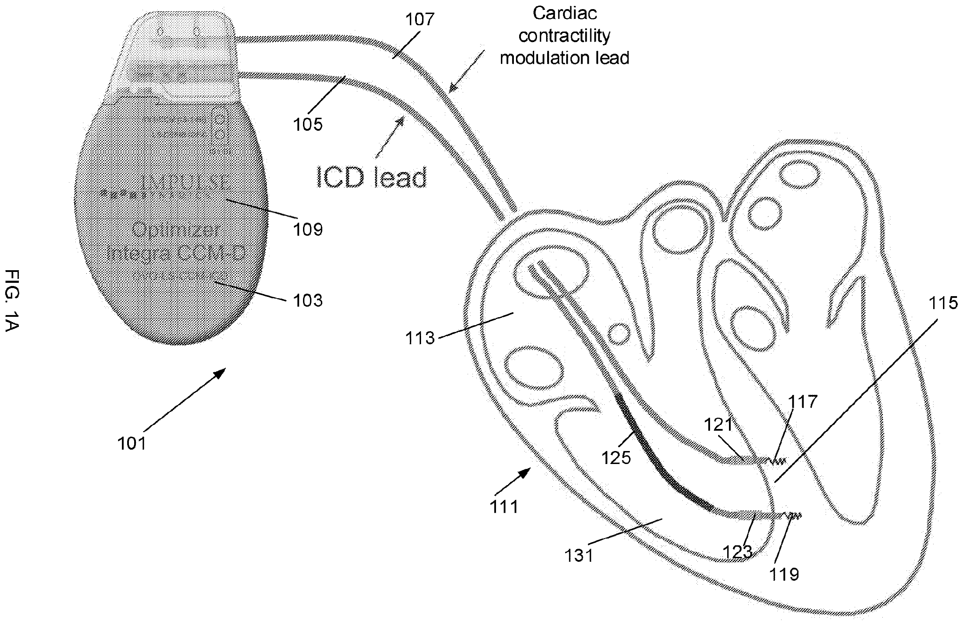

Referring now to the drawings, FIG. 1A is schematic illustration of an implantable device comprising a defibrillation lead and at least one cardiac contractility modulation lead, according to some embodiments.

In some embodiments, implantable device 101 comprises a pulse generator 103. In some embodiments, pulse generator 103 comprises a housing 109, which encases, for example: powering means (e.g. a battery), control circuitry configured for timing and generating the electrical pulses, sensing circuitry, communication circuitry, memory means, and/or other.

In some embodiments, one or more stimulation leads such as 105, 107 are connected to the housing and extend externally from it. In some embodiments, a lead comprises one or more electrical wires, surrounded by an external insulating layer. In some embodiments, a lead is comprised of two wires, having different polarities. In some embodiments, a wire of the lead is coiled.

In some embodiments, pulse generator 103 is implanted outside the heart, for example in the subclavian area. Optionally, implantation is via a minimally invasive procedure.

In some embodiments, a housing of pulse generator 103 (also referred to as "can") is implanted subcutaneously, in proximity of the left chest.

In some embodiments, leads 105 and 107 extend from pulse generator 103, and at least a distal segment of the leads is implanted within the heart 111. In some embodiments, as shown, both leads are passed through the right atrium 113, and contact, at their distal ends, the ventricular septum 115. In some embodiments, each lead contacts the septum at a different location.

It is noted that additionally or alternatively, a single lead, which includes two spaced apart stimulation electrodes, is used.

It is also noted that also in Fig. 1A both leads are shown in right ventricle 131 against ventricular septum 115, however, one or more stimulating leads may be in other locations, with consequently different effect circles and/or targeting different tissues. In some embodiments, the leads are located inside the heart, on the right side thereof optionally to take advantage of two potential advantages: a. less out-of-heart tissue being stimulated; and b. less invasive access and/or presence than in the left heart.

In some embodiments, one of the leads is implanted outside the heart, and the other lead is implanted inside the heart.

In some embodiments, each of the leads ends with a tip electrode (see 117 of lead 107, 119 of lead 105). The tip electrode may be configured as a contact electrode, a screw-in electrode, a sutured electrode, a free-floating electrode and/or other types.

In some embodiments, one or both of the leads includes a ring electrode (see 121 of lead 107, 123 of lead 105), located along the lead, proximally to the tip electrode.

In some embodiments, the ring and/or tip electrodes of the non-defibrillation lead (e.g. a pacing lead, a cardiac contractility modulation lead) are implanted in the right ventricle or in the right atria of the heart.

In some embodiments, a tip electrode is formed with a threading so as to be threaded into the tissue. Alternatively, a tip electrode is solely placed in contact with the tissue.

In some embodiments, one or both of the leads include a defibrillation coil (see 125 of lead 105). Optionally, coil 125 is located along the lead, proximally to the tip electrode and/or proximally to the ring electrode.

In some embodiments, the coil is implanted in the right ventricle, right atria or in the vena cava.

In some embodiments, lead 105 delivers defibrillation signals. In some embodiments, lead 107 delivers non-defibrillation signals, for example, a pacing signal, and/or a non-excitatory signal such as a cardiac contractility modulation signal.

In some embodiments, the cardiac contractility modulation signal is applied in contact with the ventricular tissue or within ventricular tissue.

In some embodiments, the cardiac contractility modulation signal is applied to the heart during a relative and/or absolute refractory period of the heart. In some embodiments, the signal is selected to increase the contractility of a cardiac ventricle when the electric field of the signal stimulates such ventricular tissue, for example, the left ventricle, the right ventricle and/or a ventricular septum. In some embodiments of the invention, contractility modulation is provided by phosphorylation of phospholamban caused by the signal. In some embodiments of the invention, contractility modulation is caused by a change in protein transcription and/or mRNA creation caused by the signal, optionally in the form of reversal of a fetal gene program. The term "cardiac contractility modulation " is used herein, unless otherwise noted, as a general placeholder for all such signals. It is noted that in some embodiments the cardiac contractility modulation signal may be excitatory to tissue other than that to which it is applied.

While not being limited to a single pulse sequence, the term cardiac contractility modulation is used to describe any of a family of signals which includes a significant component applied during an absolute refractory period and which has a clinically significant effect on cardiac contractility in an acute and/or chronic fashion and/or which causes a reversal of fetal gene programs and/or which increases phosphorylation of phospholamban. In some embodiments, the signal is potentially excitatory to one part of the heart but non-excitatory to other parts. For example, a signal can be excitatory in atria, but applied at a timing (relative to ventricular activation) when it is not excitatory in the ventricle.

In some embodiments of the invention, the signal while potentially stimulatory during the receptive period of the cardiac cycle is non-excitatory due to its timing. In particular, the signal is applied during the refractory period of the tissue which is affected by it and, optionally, within the absolute refractory period.

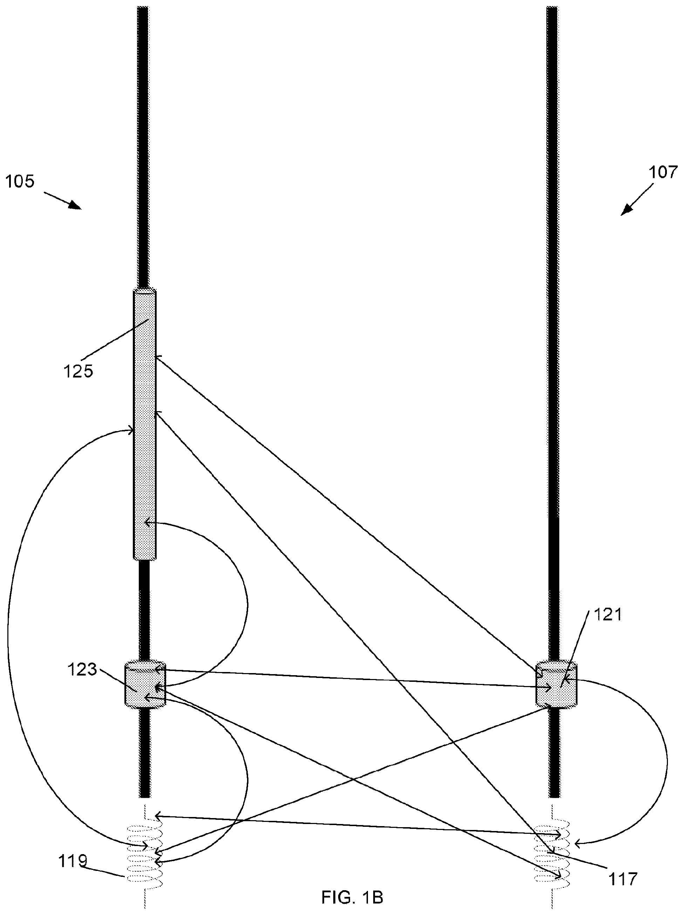

FIG. 1B schematically illustrates cross-measurements of impedance between components of two leads of an implantable device, according to some embodiments.

In some embodiments, measurement of impedance (in Ohms), as referred to herein, may include the resistance of a component or a circuit to electrical current of the test pulse. In some embodiments, the test pulse is generated by the pulse generator.

In some embodiments, impedance is measured between components (or portions) of two different leads, such as lead 105 and lead 107. Examples of these cross impedance measurements (may also be referred to as inter-lead measurements) may include:

Coil 125 and tip electrode 117; Coil 125 and ring electrode 121; Ring electrode 123 and tip electrode 117; Ring electrode 123 and ring electrode 121; Tip electrode 119 and tip electrode 117; Tip electrode 119 and ring electrode 121.

Additionally or alternatively, impedance is measured between components of a single lead. For example, in lead 105: coil 125 and ring electrode 123; coil 125 and tip electrode 119; ring electrode 123 and tip electrode 119. For example, in lead 105: tip electrode 117 and ring electrode 121.

In some embodiments, a condition of a lead component (e.g. electrode, coil and/or other electrical contact) is estimated by a two-step method, in which impedance is measured between the tested component and a first other component and then measured between the tested component and a second other component. This method may be advantageous for determining which of the components was the cause of a deviation from a normal and/or expected impedance range.

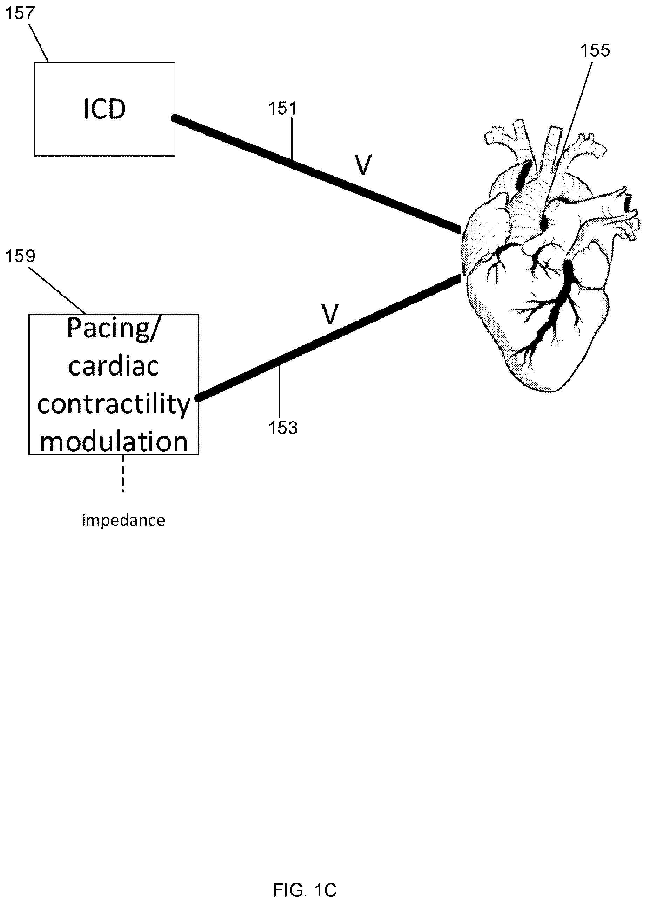

FIG. 1C is a schematic diagram of impedance measurements in a cardiac device comprising two leads, according to some embodiments.

In some embodiments, the device comprises two stimulation leads 151, 153 implanted in the heart 155. In some embodiments, stimulation lead 151 is configured for applying defibrillation signals, being connected to ICD module 157. In some embodiments, stimulation lead 153 is configured for applying non-defibrillation signals, such as cardiac contractility modulation and/or pacing signals, being connected to pacing/cardiac contractility modulation module 159.

In some embodiments, impedance is measured for a single lead, e.g. between components (coil, tip electrode, ring electrode) of a single lead. Additionally or alternatively, cross-impedance is measured between the two leads. In some embodiments, impedance is estimated by calculating resistance of relevant circuitry. For example, for estimating impedance of the ICD lead alone, it is possible to measure impedance for the pacing/cardiac contractility modulation lead; then measure cross-impedance between the two leads; then subtract the relevant resistance of the circuitry, e.g. as measured for the pacing/cardiac contractility modulation lead alone to estimate the ICD lead impedance.

In some embodiments, impedance is measured between a coil and a first electrode, and then between the coil and a second electrode and/or between the first and second electrodes. In some embodiments, impedance between the two electrodes is subtracted from a sum of impedances between the coil and two electrodes. By dividing the result by two, the coil impedance may be assessed.

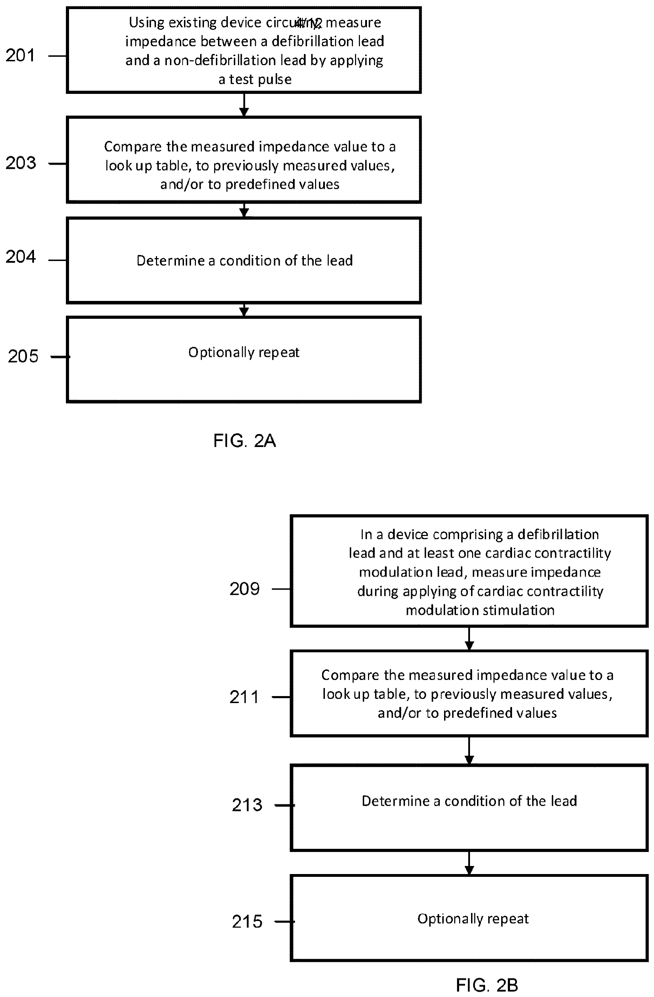

FIGs. 2A-D are flowcharts of general methods for assessing a lead condition by measuring impedance in a cardiac device, according to some embodiments. In some embodiments, the cardiac device comprises at least one defibrillation lead and at least one non-defibrillation lead, e.g. a pacing lead, a cardiac contractility modulation lead, and/or other.

The flowchart of FIG. 2A describes a method for assessing lead integrity using existing device circuitry, in accordance with some embodiments.

The leads of an active implanted cardiac device are, in some cases, most prone to failure, for example as compared to other components of the device. Lead failures or defects, such as a cut-off in the lead wire or a hole in the lead insulation sheath may form due to tension on the lead, body movements, blood flow in contact with the lead, and/or other. In some cases, when two or more leads are positioned in a blood vessel or in a body lumen, contact between the two leads may increase a likelihood of lead failure. Therefore, in some cases, for maintaining a safe and functioning system it is desired to provide for quick and efficient detection of a lead condition (e.g. fracture, dislodgment, connectivity problem, insulation problem, deformation or the like).

In some embodiments, impedance measurements may allow for detecting the condition of the lead, due to that the measured impedance value is affected by defects (such as mentioned above) and deviates from an expected or normal value or range. Based on this change in impedance value and/or based on an absolute impedance value, existence of a defect can be identified. In an example, an insulation break may expose the wires of the lead to fluid and/or blood, causing, for example, a reduction in the measured value as compared to an expected (and/or previously measured) value. In another example, a cut-off and/or narrowing in the lead wire may result in a higher impedance value. In another example, fluctuations in measured impedance values may indicate a dynamic, ongoing situation in which the lead sometimes functions normally and in other times does not. In some cases, the fluctuations in impedance are caused as a result of a change of body posture, movement, breathing and/or heart pulsation, which may move the lead(s) and/or associated circuitry.

In an exemplary configuration of a device including a defibrillation lead and a non-defibrillation lead. At 201, in some embodiments, impedance is measured between the two leads by applying a test pulse. In some embodiments, the impedance is measured via existing device circuitry, using no additional circuity or components. In an example, as further detailed below, current across a grounded resistor connecting the non-defibrillation lead is measured for determining the impedance between the two leads.

At 203, in accordance with some embodiments, the measured impedance value is compared to one or more of: a look up table, one or more previously measured values, one or more predefined values (e.g. values set by manufacturer, values set by an operator (e.g. physician, technician), values known from literature, baseline values, and/or others).

In some embodiments, a baseline measurement of impedance between one or more sets of components of the device leads is performed immediately following implantation. Additionally or alternatively, a baseline measurement is performed during a follow up visit, optionally to update previously measured baseline values. In some embodiments, a baseline measurement is performed at any time in which a condition of the device and its components is known to be normal and properly functioning.

In some embodiments, a measured change in impedance value which is more than 20%, more than 30%, more than 40%, more than 50%or intermediate, larger or smaller percentage different (i.e. lower or higher) than the baseline value indicates a defect. In some embodiments, a measured change in impedance value which is less than 20% different (i.e. lower or higher) than the baseline value indicates a normal condition. In some embodiments, a reduction in impedance value indicates shorted circuitry. In some embodiments, a rise in impedance value indicates disconnection.

In an example, a baseline ring electrode-to-coil impedance value is 250 ohm. In case a later measured ring electrode-to-coil impedance value increases to, for example, 500 ohm, this may indicate a short circuit, either in the coil lead (defibrillation lead) or in the electrode lead (non-defibrillation lead).

In some embodiments, a controller of the device is programmed to carry out the comparison of a measured impedance value to an expected impedance value. Additionally or alternatively, a remote server, computer program, and/or cellular phone application in communication with the device are configured carry out the comparison.

At 204, in accordance with some embodiments, a condition of the lead is determined based on the results of the comparison. In some embodiments, the measured impedance value indicates existence of a defect if it is, for example, higher than a set threshold, lower than a set threshold, not within a desired or predefined range. Optionally, if a defect is detected, an alert is issued. In some embodiments, the alert is issued (e.g. via wireless communication) from the device controller to the physician and/or other supervising authority. In some embodiments, if the impedance test is performed during a routine device check (e.g. at a technician lab and/or physician office), the defected lead may be replaced. In some embodiments, if a defect is detected in the defibrillation coil and/or its connections, the defibrillation coil (and/or the defected connection) is immediately replaced.

At 205, optionally, the impedance measurement is repeated. In some embodiments, assuming no defect has been detected, the measurement is performed periodically, for example every 30 seconds, 1 minute, 5 minutes, 15 minutes, 1 hour, 2 hours, 4 hours, 12 hours, 24 hours, 2 days, 5 days, 1 week or intermediate, longer or shorter time intervals.

In some embodiments, if the resulting impedance of a consecutive measurement varies from the previous measurement, for example by more than 5%, more than 10%, more than 20% or intermediate, larger or smaller percentage, this may indicate a problem, optionally in lead insulation. In some embodiments, an alert is provided when change above a threshold is identified.

In some embodiments, assessment of which lead (or lead component, e.g. an electrode) has the defect, one or more additional measurements may be performed, optionally using an additional (e.g. second) lead as reference.

Additionally or alternatively, the measurement is performed at a follow-up visit to the clinic, for example, every month, every 2 months, every 6 months, every year, or intermediate, longer or shorter time intervals.

**FIG. 2B** is a flowchart of a method for measuring impedance during applying of a cardiac contractility modulation stimulation, according to some embodiments.

In an exemplary configuration, the device comprises a defibrillation lead and a second lead, which applies cardiac contractility modulation stimulation. At 209, in some embodiments, impedance is measured between the two leads, for example as described herein. Optionally, impedance is measured between the coil of the defibrillation lead and one of the electrodes of the cardiac contractility modulation lead (e.g. tip electrode, ring electrode). A potential advantage of measuring between these components may include avoiding conduction of current through a different path such as between the coil of the defibrillation lead and the pulse generator itself (coil-to-CAN), thereby avoiding passing current at a location of sensory nerves.

In some embodiments, impedance measurement is performed during applying of the cardiac contractility modulation signal. Optionally, the measurement is performed during an initial phase of the cardiac contractility modulation signal. For example, if a duration of the cardiac contractility modulation signal is 20ms-30ms long, the impedance measurement is performed at, for example, at 10 μsec, 30 μsec, 120 μsec, 2 msec, 5 msec, 7 msec or intermediate, later or earlier time into the cardiac contractility modulation signal. Additionally or alternatively, the impedance measurement is performed at a later phase of the cardiac contractility modulation signal, for example at 10msec, 15sec, 20msec or intermediate, later or earlier time into the cardiac contractility modulation signal.

In some embodiments, a duration of a test pulse for measuring impedance is as short as possible, yet still sufficiently long so as to provide a reasonable estimate of the measured impedance. Optionally, the duration of the test pulse is set according to the specific circuitry involved.

In some embodiments, in which the cardiac contractility modulation lead "splits" into two spaced apart stimulation electrodes, the measurement can be performed between the defibrillation coil and each of the electrodes. Optionally, if a certain measurement path (e.g. between coil and a first electrode) causes pain and/or otherwise sensed by the patient, the second path (e.g. between coil and the second electrode) may be selected.

In some embodiments, in which the non-defibrillation lead acts as a pacing lead, the impedance measurement may be performed during the pacing signal. In some embodiments, a duration of the pacing signal is between 0.1 ms-1.5ms, 0.2 ms- 1 ms, 0.5 ms- 1.2 ms, or intermediate, longer or shorter duration.

At 211, in accordance with some embodiments, the measured impedance value is compared to one or more of: a look up table, one or more previously measured values, one or more predefined values (e.g. values set by manufacturer, values set by an operator (e.g. physician, technician), values known from literature, baseline values, and/or others), for example as described hereinabove. At 213, in accordance with some embodiments, a condition of the lead is assessed, for example as described hereinabove. At 215, optionally, the measurement is repeated.

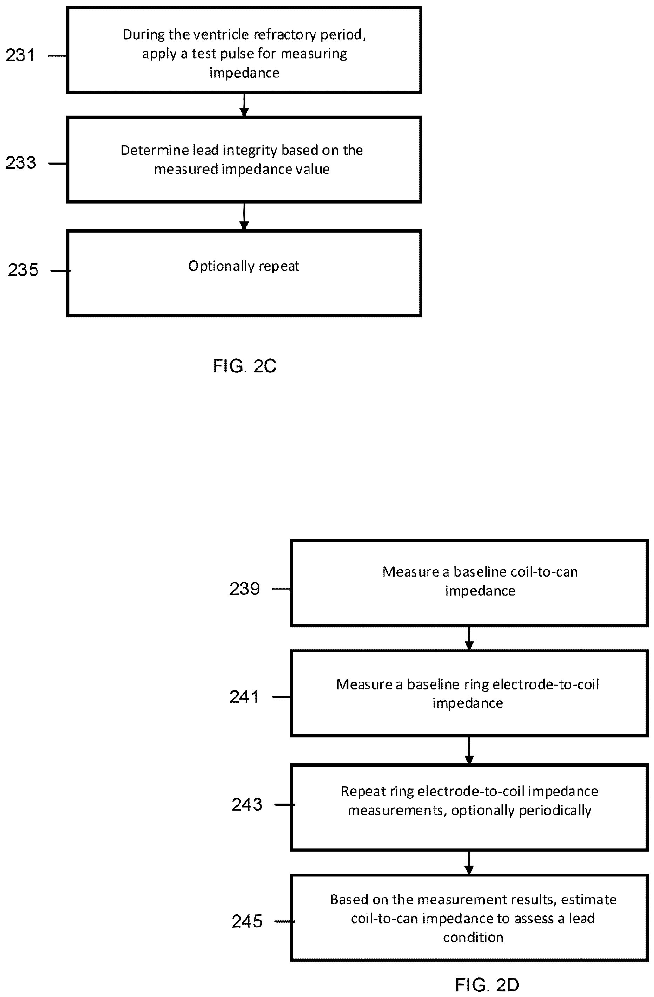

**FIG. 2C** is a flowchart of a method for measuring during the ventricle refectory period, according to some embodiments. At 231, in accordance with some embodiments, a test pulse for measuring impedance is applied during the ventricle refectory period. Optionally, the test pulse is applied during the absolute ventricle refractory period. A potential advantage of measuring impedance during the ventricle refractory period may include that during that time, the cardiac cell is unable to initiate another action potential, therefore a risk of generating undesired contraction of the heart by the test pulse is reduced or prevented.

At 233, in accordance with some embodiments, lead integrity is determined based on the measured impedance value, for example as described hereinabove. At 235, in accordance with some embodiments, the impedance measurement is repeated. Optionally, the measurement is repeated during one or more successive ventricle refractory periods of the cardiac cycle.

**FIG. 2D** is a flowchart of a method for assessing lead integrity by a non-direct impedance measurement, according to some embodiments. In some embodiments, the method is performed for estimating an ICD lead integrity by calculating or estimating coil-to-can impedance. In some embodiments, the method is applied in attempt to reduce the number of times in which impedance is measured via a path, which may cross-innervated tissue, such as the coil-to-can path.

At 239, in accordance with some embodiments, a baseline coil-to-can impedance value is measured (such as between the coil and the pulse generator housing).

At 241, in accordance with some embodiments, a baseline ring electrode-to-coil impedance is measured (such as between the ring electrode (of the non-defibrillation lead), and the coil of the defibrillation lead).

In some embodiments, the baseline measurements of 239 and/or 241 are each performed only once, or performed several times at relatively long time intervals. For example, once every 2-6 months, once every 6-24 weeks, once every 6-12 months, or intermediate longer or shorter time intervals. Optionally, these measurements are performed during a visit to the clinic.

At 243, in accordance with some embodiments, the ring electrode-to-coil impedance is measured again. Optionally, this measurement is performed periodically. In some embodiments, this measurement is repeated at shorter time intervals, for example every 30 minutes, every hour, every 4 hours, every day, every week, or intermediate, longer or shorter time periods.

At 245, in accordance with some embodiments, using the baseline coil-to-can measurement and using differences between a currently measured ring electrode-to-coil impedance versus the baseline measured ring electrode-to-coil impedance, a current coil-to-impedance is estimated.

In some embodiments, the current coil-to-can impedance is calculated or estimated using the following function: Coil-to-Can impedance (t)= Coil-to-Can impedance (baseline)*electrode-to-coil impedance (t) / electrode-to-coil impedance (baseline). It is noted that other functions, formulas, factors (e.g. linearizing factors) and/or others may be applied. Optionally, the specific parameters for calculating or estimating a current impedance (such as coil-to-can impedance) are selected according to the specific device circuitry.

In some embodiments, a condition of the lead is assessed based on the estimated impedance. Optionally, an integrity of the defibrillation lead and/or components thereof, such as the coil, are assessed.

A potential advantage of the method of FIG. 2D is that during normal operation, the defibrillation lead does not apply as many stimulations as the non-defibrillation lead, therefore it may be hard to detect any defects which may have formed over time on the defibrillation lead. Using the above-described method, the condition of the defibrillation lead may be estimated more often. Another potential advantage may include substantially avoiding the need for applying a test pulse to the coil-to-can path directly, thereby avoiding a path, which may cause pain to the patient, due to innervated tissue along the way.

In some embodiments, a test pulse applied for measuring coil-to-can impedance is shorter in duration and applied at a higher voltage as compared to a test pulse applied for measuring electrode-to-coil impedance, which can be of lower voltage and optionally a longer duration.

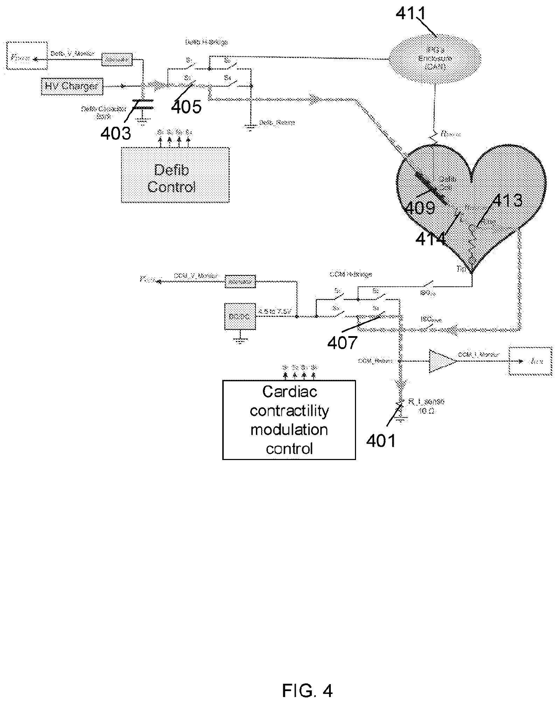

FIG. 4 is a schematic diagram of circuitry suitable for impedance measurement in a device comprising a defibrillation lead and at least one cardiac contractility modulation lead, according to some embodiments.

In some embodiments, by measuring current ("Iccm") flowing via the cardiac contractility modulation return path, for example, via grounded resistor "R_I_sense" 401, impedance is assessed.

In some embodiments, by measuring current flow as opposed to, for example, measuring a change in a capacitor's capacitance, a lower test pulse voltage, for example between 0-8 V, 0-5 V, 0.1-3 V, 0.1-8 V or intermediate, higher or lower voltage can be applied, for example as compared to the higher voltage level needed for generating a noticeable and measureable change in the capacitor, such as voltage between 8-16 V, 10-16 V, 12-16 V, 14-16 V or intermediate, higher or lower voltage.

In some embodiments, as shown in the diagram of FIG. 4, the device comprises two modules: a defibrillation module, including for example an H-bridge configuration of switches for controlling conduction of current to the defibrillation lead, and a capacitor bank for setting voltage to the defibrillation coil; and a cardiac contractility modulation module, including for example an H-bridge configuration of switches for controlling conduction of current to the cardiac contractility modulation lead, and a DC power source.

The following describes an example of impedance estimation using the shown circuitry: in some embodiments, the defibrillation capacitor bank 403 is charged, for example to a voltage between 8-16 V, for applying a pulse. In some embodiments, switch S3 (see 405) of the defibrillation H-bridge and switch S4 (see 407) of the cardiac contractility modulation H-bridge are closed simultaneously for a short period of time for applying of the pulse, for example 5 $\mu$s, 10 $\mu$s, 15 $\mu$s, 20 $\mu$s or intermediate, shorter or longer time periods. Optionally, closing of the switches is performed shortly after an R-wave of the cardiac cycle, optionally during the total ventricle refractory period, for example, less than 50 msec, less than 20 msec, less than 10 msec, less than 5 msec, 4 msec, less than 3 msec, less than 2 msec or intermediate, longer or shorter time periods following the R-wave.

In some embodiments, a duration of the pulse is selected to be long enough so that an analog to digital converter of the device (not shown) may correctly sample the current flowing through the cardiac contractility modulation return path-"Iccm". Optionally, the Iccm current is measured as the current across the grounded resistor 401. A potential advantage of measuring the impedance by measuring Iccm on the grounded resistor may include that the pulse can be applied at a relatively low voltage, e.g. between 0-8 V, 0.1-7 V, 0.5-8.5 V or intermediate, higher or lower voltage, due to the relatively high sensitivity of this measurement, for example as compared to measuring a change in capacitance. Using a pulse of relatively low voltage may imply less pain and/or sensation to the patient.

Additionally or alternatively, in some embodiments, the voltage across the defibrillation capacitor bank 403 ("Vdefib") is sampled.

In some examples, an estimation of the impedance between the defibrillation coil 409 and the device housing 411 (coil-to-CAN) cannot be deduced directly from the above measurements (i.e. of Iccm). This may be due to the fact that a connection 414 (e.g. a lead wire) extending between the coil 409 and the ring electrode 413 is in many cases coiled, and imposes its own impedance (typically ranging in tens of Ohms). In some cases, an impedance of the coil itself varies (optionally in a non-linear manner) with the flow of current, resulting in that for different values of voltage, different impedance measures are obtained.

Another factor which may affect estimation of the coil-to-can impedance directly from the measurements of Iccm may include that S3 of the defibrillation bridge may present an impedance which varies non-linearly with the flow of current. This may interfere with assessment of direct current.

In view of the above, in some embodiments, coil-to-can impedance is estimated for example as described above in FIG. 2D. Optionally, a baseline measurement of the coil-to-can impedance is performed (e.g. using a method for example as described for the circuitry of FIG. 3). Then, using an equation or a function, a look up table, the device specifications, and/or other reference, a linearizing factor is calculated. In some embodiments, the linearizing factor ties Iccm with the one time measurement of the coil-to-can impedance. In some embodiments, for reaching an estimated coil-to-can impedance, for example in future measurements in which the coil-to-can measurement itself is not performed, but Iccm is measured, the linearizing factor may be applied. In an example, the estimated coil-to-can impedance is calculated as follows:

$$\text{Estimated coil-to-can impedance} = f\ (\text{Iccm, baseline coil-to-can } \Omega)$$

In some embodiments, one or more thresholds are set for determining when the resulting estimated coil-to-can impedance indicates a normal, non-damaged condition of the defibrillation lead; and when the estimated coil-to-can impedance may indicate a defect in the integrity of the defibrillation lead.

In an example, thresholds are set as follows:

Inappropriately LOW impedance if Estimated Impedance < 25 $\Omega$

Impedance OK if 25 $\Omega$ $\leq$ Estimated Impedance $\leq$ 100$\Omega$

Inappropriately HIGH impedance if Estimated Impedance > 100 $\Omega$

In some embodiments, when impedance is found not to be within an expected range, an alert and/or other notification are generated.

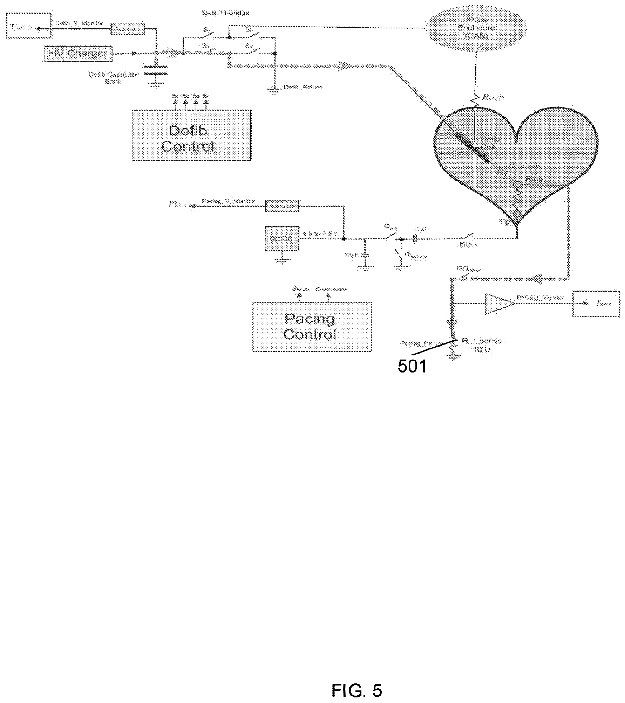

FIG. 5 is a schematic diagram of circuitry suitable for impedance measurement in a device comprising a defibrillation lead and at least one pacing lead, according to some embodiments.

In this diagram, the device comprises a defibrillation module, for example as described above in FIG. 4, and a pacing module, for controlling activation of a pacing lead.

In a similar manner to the method described above in FIG. 4, impedance may be assessed via measuring "Ipace", the current across grounded resistor 501, and/or by measuring Vdefib, for example as described in FIG. 4.

In some embodiments, one of the differences between the circuitry described in FIG. 4 and the circuitry described in FIG. 5 is that a pulse applied by the non-defibrillation lead (e.g. a cardiac contractility modulation lead in FIG. 4; and a pacing lead in FIG. 5) has different properties, such as amplitude, duration, timing of the pulse.

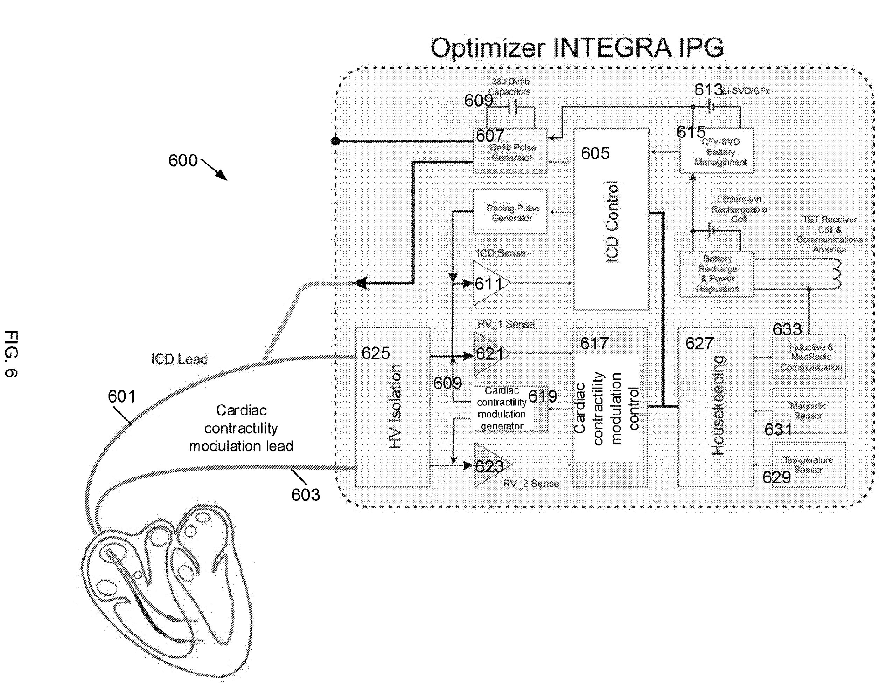

FIG. 6 is a schematic diagram showing components of an implantable ICD/cardiac contractility modulation device 600, according to some embodiments.

In some embodiments, the device comprises an ICD lead 601, and a cardiac contractility modulation lead 603.

In some embodiments, activation of the ICD lead is by an ICD module which includes or is connected to: ICD control 605, a defibrillation pulse generator 607 (via one or more capacitors 609), a power source (e.g. a battery 613) and power source management circuitry 615, and ICD sense 611 which senses an applied pulse to verify the pulse is within a selected (e.g. programmed) amplitude and/or duration. In some embodiments, activation of the one or more cardiac contractility modulation leads 621, 623, which are optionally positioned in the right ventricle, is by a cardiac contractility modulation module, which includes or is connected to: cardiac contractility modulation control 617, a cardiac contractility modulation generator 619.

It is noted that in some embodiments, the ICD coil and one more electrodes for pacing and/or cardiac contractility modulation are configured on the same lead.

In some embodiments, the leads are connected to isolation 625.

In some embodiments, the device comprises a housekeeping module 627, which includes or is connected to one or more sensor such as a temperature sensor 629, a magnetic sensor 631, and communication means 633 such as an antennae, a receiver and the like. Other sensors may include flow sensors, pressure sensors, acceleration sensors, and/or other.

In some embodiments, data received from the one or more sensors is received as input. Optionally, the input is processed by the device control (e.g. by the ICD control, cardiac contractility modulation control, and/or a general controller, not shown) and is optionally used as input to decision making processes in device 600.

In some embodiments, the device control (e.g. the ICD control, cardiac contractility modulation control, and/or a general controller, not shown) executes one or more logics to decide, for example, a timing and/or other parameters of a signal and/or if a signal is to be applied.

A memory (not shown) is optionally provided, for example, to store logic, past effects, therapeutic plan, adverse events and/or pulse parameters.

A logger (not shown) is optionally provided to store activities of device 600 and/or of the patient. Such a log and/or programming may use a communication module 633 to send data from device 600, for example, to a programmer (not shown) and/or to receive data, for example, programming, for example, pulse parameters.

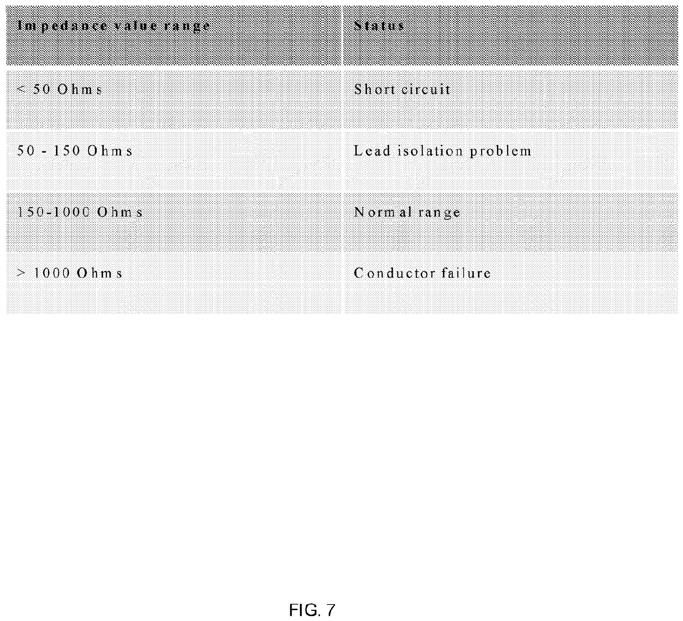

FIG. 7 is an example of an impedance look up table for assessing a lead condition, according to some embodiments.

In some embodiments, an existence and optionally type of lead condition is determined according to the measured impedance value. Optionally, the device (e.g. the device controller) is programmed with ranges and/or thresholds according to which an alert is generated if a lead condition is detected. The table of FIG. 7 shows examples of several thresholds and ranges, each associated with a different type of lead condition. It is noted that this table is only an example and that values, thresholds, ranges and/or lead conditions other than the ones shown may be used.

| Impedance value range | Status |
| --- | --- |
| < 50 Ohms | Short circuit |
| 50 - 150 Ohms | Lead isolation problem |
| 150-1000 Ohms | Normal range |
| > 1000 Ohms | Conductor failure |

FIG. 8 is a flowchart of a method for estimating a connectivity condition in a device comprising a defibrillation lead and two pacing leads, according to some embodiments.

In some embodiments, in a device configuration which includes three leads or 3 different components (e.g. an ICD lead and two electrodes, such as two pacing electrodes; an ICD lead an two cardiac contractility modulation electrodes; an ICD lead and a cardiac contractility modulation or pacing lead having a "split" end with separated electrodes), cross impedance measurements may provide for identifying lead condition and/or connectivity problems.

In the example of FIG. 8, cross impedance measurements in an ICD/pacing device are described. It is noted that a similar method may be applied to an ICD/cardiac contractility modulation device.

At 801, in accordance with some embodiments, an ICD coil and two electrodes such as two pacing electrodes are implanted.

At 803, in accordance with some embodiments, impedance measurement is performed between the ICD coil and a first electrode. At 805, in accordance with some embodiments, the result of the measurement is assessed to determine whether the received impedance value indicates a normal condition of the lead, or whether the received impedance value indicates a connectivity/integrity problem. If the impedance value is within the expected (normal) range, at 807, in accordance with some embodiments, a repeated measurement may be performed after waiting a set time, for example 3 seconds, 5 seconds, 10 seconds, 30 seconds, 60 seconds, 120 seconds, 360 seconds or intermediate, longer or shorter time periods.

Alternatively, if the received impedance value indicates a problem, at 809, in accordance with some embodiments, an impedance measurement is performed between the first and second electrodes. Additionally or alternatively, at 811, an impedance measurement between the ICD coil and the second electrode is performed.

At 813 and 815, the measured value is checked whether it falls within an expected range.

If the impedance value measured between the first and second electrodes is within the expected the range, this suggests that the defect is in the ICD coil, therefore at 817 an alert is issued regarding the ICD coil status. If the impedance value measured between the first and second electrodes is not within the expected range, this suggests that the defect is in the first electrode, therefore at 819 an alert is issued regarding the first electrode status.

If the impedance value measured between the ICD coil and the second electrode is within the expected range, this suggests that the defect is in the first electrode, therefore at 819 an alert is issued regarding the first electrode status. If the impedance measured between the ICD coil and the second electrode is not within the expected range, this suggests that the defect is in the ICD coil, therefore at 817 an alert is issued regarding the ICD coil status.

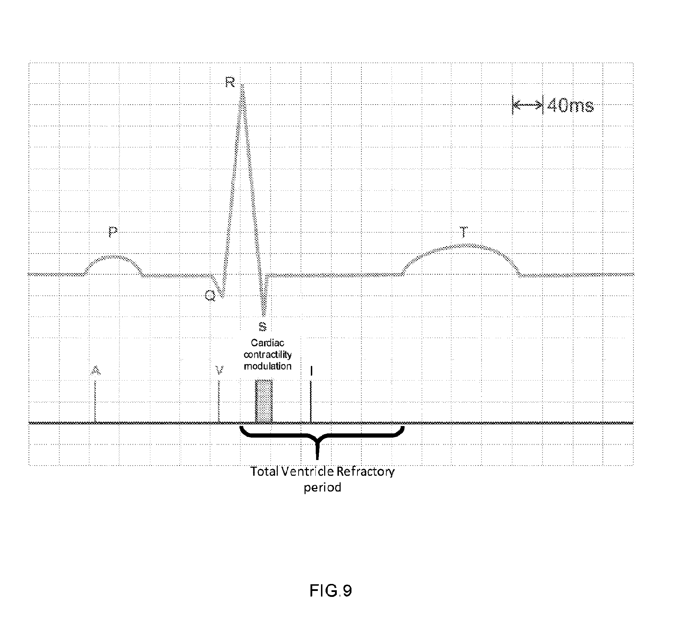

FIG. 9 is a graphical representation of a cardiac cycle showing the relative timing of applying of a cardiac contractility modulation stimulation and of a test pulse for impedance measurement, according to some embodiments.

In some embodiments, a test pulse for measuring impedance is applied during the total ventricle refractory period, which normally occurs between a timing of the R wave and the T wave of the cardiac cycle. Normally, the total refractory period is between 200-400 msec long.

In some embodiments, the applied test pulse duration (indicated "I" in the graph) is between 1-100 $\mu$sec, e.g. 10 $\mu$sec, 15 $\mu$sec, 30 $\mu$sec, 50 $\mu$sec, 70 $\mu$sec, or intermediate, longer or shorter duration.

Optionally, in some embodiments, the impedance measurement is performed during applying of a cardiac contractility modulation signal, using the cardiac contractility modulation signal itself as the test pulse. In such situation, the applied pulse may be between 1msec-50 msec long, e.g. 5 msec, 10 msec, 30 msec, 40 msec or intermediate, longer or shorter duration. Alternatively, the impedance measurement is during the ventricle refractory period but not when applying the cardiac contractility modulation signal, for example, before or after applying the cardiac contractility modulation signal. When not using the cardiac contractility modulation signal as the test pulse, a lower voltage (i.e. with respect to the voltage applied for cardiac contractility modulation ) may be used. Optionally, the test pulse voltage is selected above a threshold high enough for generating by the device and for resulting in a measurable (detectable) current. In an example, test pulse voltage is above 0.01V, above 0.05V, above 0.1 V or intermediate, higher or lower voltage. In some embodiments, optionally in addition to a lower threshold, an upper voltage threshold is selected, for example of 0.1V, 0.5 V, 1V, or intermediate, higher or lower voltage.

It is noted that these times can change between hearts and also under different conditions, such as pharmaceutical intake, anatomic excitation level, heart rate, recent arrhythmia and/or exercise. In some embodiments, the pulse generator is pre-programmed with parameters that take such refractory periods into account. Optionally, different numbers are used (e.g. stored in a device memory) for different conditions (e.g., different heart rates).

The terms "comprises", "comprising", "includes", "including", "having" and their conjugates mean "including but not limited to".

The term "consisting of" means "including and limited to".

The term "consisting essentially of" means that the composition, method or structure may include additional ingredients, steps and/or parts, but only if the additional ingredients, steps and/or parts do not materially alter the basic and novel characteristics of the claimed composition, method or structure.

As used herein, the singular form "a", "an" and "the" include plural references unless the context clearly dictates otherwise. For example, the term "a compound" or "at least one compound" may include a plurality of compounds, including mixtures thereof.

Throughout this application, various embodiments of this invention may be presented in a range format. It should be understood that the description in range format is merely for convenience and brevity and should not be construed as an inflexible limitation on the scope of the invention. Accordingly, the description of a range should be considered to have specifically disclosed all the possible subranges as well as individual numerical values within that range. For example, description of a range such as from 1 to 6 should be considered to have specifically disclosed subranges such as from 1 to 3, from 1 to 4, from 1 to 5, from 2 to 4, from 2 to 6, from 3 to 6 etc., as well as individual numbers within that range, for example, 1, 2, 3, 4, 5, and 6. This applies regardless of the breadth of the range.

Whenever a numerical range is indicated herein, it is meant to include any cited numeral (fractional or integral) within the indicated range. The phrases "ranging/ranges between" a first indicate number and a second indicate number and "ranging/ranges from" a first indicate number "to" a second indicate number are used herein interchangeably and are meant to include the first and second indicated numbers and all the fractional and integral numerals therebetween.

As used herein the term "method" refers to manners, means, techniques and procedures for accomplishing a given task including, but not limited to, those manners, means, techniques and procedures either known to, or readily developed from known manners, means, techniques and procedures by practitioners of the chemical, pharmacological, biological, biochemical and medical arts.

As used herein, the term "treating" includes abrogating, substantially inhibiting, slowing or reversing the progression of a condition, substantially ameliorating clinical or aesthetical symptoms of a condition or substantially preventing the appearance of clinical or aesthetical symptoms of a condition.

It is appreciated that certain features of the invention, which are, for clarity, described in the context of separate embodiments, may also be provided in combination in a single embodiment. Conversely, various features of the invention, which are, for brevity, described in the context of a single embodiment, may also be provided separately or in any suitable subcombination or as suitable in any other described embodiment of the invention. Certain features described in the context of various embodiments are not to be considered essential features of those embodiments, unless the embodiment is inoperative without those elements.

Although the invention has been described in conjunction with specific embodiments thereof, it is evident that many alternatives, modifications and variations will be apparent to those skilled in the art. Accordingly, it is intended to embrace all such alternatives, modifications and variations that fall within the spirit and broad scope of the appended claims.

All publications, patents and patent applications mentioned in this specification are herein incorporated in their entirety by reference into the specification, to the same extent as if each individual publication, patent or patent application was specifically and individually indicated to be incorporated herein by reference. In addition, citation or identification of any reference in this application shall not be construed as an admission that such reference is available as prior art to the present invention. To the extent that section headings are used, they should not be construed as necessarily limiting. In addition, any priority document(s) of this application is/are hereby incorporated herein by reference in its/their entirety.

The present disclosure further relates to the following aspects:

A first aspect of the present disclosure relates to a method of testing a lead condition in an implanted cardiac device comprising a first defibrillation lead and a second non-defibrillation lead, the method comprising: measuring impedance between said first defibrillation lead and said second non-defibrillation lead by applying a test pulse; and determining a condition of at least one of said defibrillation lead and said non-defibrillation lead according to the measured impedance value.

A second aspect of the present disclosure, as a variant of the first aspect, relates to a method, wherein a timing of said applying of said test pulse is selected so as not to stimulate contraction.

A third aspect of the present disclosure, as a variant of any one of the first or second aspect, relates to a method, wherein a duration of said test pulse is short enough so as to avoid or reduce pain caused by said test pulse to a patient in which said cardiac device is implanted.

A fourth aspect of the present disclosure, as a variant of any one of the first to third aspect, relates to a method, wherein said second non-defibrillation lead is configured for applying cardiac contractility modulation stimulation.

A fifth aspect of the present disclosure, as a variant of the fourth aspect, relates to a method, wherein said applying a test pulse is during applying of the cardiac contractility modulation stimulation.

A sixth aspect of the present disclosure, as a variant of the fourth aspect, relates to a method, wherein said applying a test pulse is during a ventricle refractory period, before or after applying of the cardiac contractility modulation stimulation.

A seventh aspect of the present disclosure, as a variant of any one of the first to sixth aspect, relates to a method, wherein said defibrillation lead comprises a coil, and said non-defibrillation lead comprises a ring electrode and a tip electrode; said measuring impedance comprising measuring impedance between at least one of: said coil and said ring electrode; said coil and said tip electrode.

An eighth aspect of the present disclosure, as a variant of any one of the first to seventh aspect, relates to a method, wherein said determining a condition of at least one of said defibrillation lead and said non-defibrillation lead is by one or more of: comparing said impedance value to one or more of: a look-up table; one or more previously measured values; one or more predefined values.

A ninth aspect of the present disclosure, as a variant of any one of the first to eighth aspect, relates to a method, comprising repeating said measuring once a day.

A tenth aspect of the present disclosure, as a variant of any one of the first to ninth aspect, relates to a method, comprising issuing an alert regarding said lead condition.

An eleventh aspect of the present disclosure, as a variant of any one of the first to tenth aspect, relates to a method, wherein said determining a lead condition comprises determining at least one of: lead fracture, dislodgment of the lead, lead insulation, lead connectivity, lead deformation.

A twelfth aspect of the present disclosure, as a variant of any one of the first to eleventh aspect, relates to a method, wherein at least a portion of said first lead and at least a portion of said second lead is implanted in contact with a wall of the right ventricle of the heart.

A thirteenth aspect of the present disclosure, as a variant of any one of the first to twelfth aspect, relates to a method, wherein said defibrillation lead comprises a coil, ring electrode and tip electrode; and said non-defibrillation lead comprises a ring electrode and a tip electrode; said measuring impedance comprising measuring inter-lead impedance between one of said coil, ring electrode and tip electrode of said defibrillation lead and one of said ring electrode and tip electrode of said non-defibrillation lead.

A fourteenth aspect of the present disclosure, as a variant of any one of the first to thirteenth aspect, relates to a method, wherein said non-defibrillation lead comprises a cardiac contractility modulation (cardiac contractility modulation) lead or a pacing lead.

A fifteenth aspect of the present disclosure relates to an implantable cardiac device comprising: a first defibrillation lead including a coil, a ring electrode and a tip electrode; a second non-defibrillation lead including a ring electrode and a tip electrode; and circuitry for controlling and activating said coil and electrodes, said circuitry including at least one grounded resistor electrically connected to said second non-defibrillation lead; said circuitry configured to measure current across said grounded resistor in response to an applied test pulse to obtain an indication of a condition of at least said first defibrillation lead or a portion thereof.

A sixteenth aspect of the present disclosure, as a variant of the fifteenth aspect, relates to a device, wherein said circuitry is at least partially disposed within a housing of said cardiac device, said leads extending from within said housing.

A seventeenth aspect of the present disclosure, as a variant of any one of the fifteenth to sixteenth aspect, relates to a device, wherein said current measured across said grounded resistor in response to an applied test pulse is used for calculating impedance between said coil and said device housing.

An eighteenth aspect of the present disclosure, as a variant of the seventeenth aspect, relates to a device, wherein said circuitry is configured to calculate said impedance between said coil and device housing using a pre-determined linearizing factor.

A nineteenth aspect of the present disclosure, as a variant of any one of the fifteenth to eighteenth aspect, relates to a device, wherein said circuitry is programmed for timing said test pulse during an expected total refractory period of the cardiac cycle.

A twentieth aspect of the present disclosure, as a variant of any one of the fifteenth to nineteenth aspect, relates to a device, wherein said non-defibrillation lead is a cardiac contractility modulation lead, and said circuitry is programmed for timing said measurement of current on said grounded resistor during applying of a cardiac contractility modulation signal.

A twenty-first aspect of the present disclosure relates to a method for assessing a lead condition in an implantable cardiac device including a defibrillation lead having a coil and at least one non-defibrillation lead having at least one electrode, the method comprising: applying a test pulse to measure a baseline impedance between the defibrillation coil and the device housing; applying, a test pulse to measure a baseline impedance between the electrode of the non-defibrillation lead and the defibrillation coil; applying, periodically, a test pulse to measure impedance between the electrode of the non-defibrillation lead and the defibrillation coil; and estimating, according to a difference between a currently measured impedance level measured at said third applying and one or both of said baseline measurements, a current impedance between the coil and said device housing to assess a condition of said defibrillation lead.

A twenty-second aspect of the present disclosure, as a variant of the twenty-first aspect, relates to a method, wherein said third applying is performed more frequently than said first and second applying.

A twenty-third aspect of the present disclosure, as a variant of any one of the twenty-first to twenty-second aspect, relates to a method, wherein said applying periodically comprises applying every 24 hours.

A twenty-fourth aspect of the present disclosure relates to an implantable cardiac device comprising: a first defibrillation lead; a second non-defibrillation lead; circuitry for controlling said leads and for measuring impedance, said circuitry configured to apply a test pulse to measure impedance between said first defibrillation lead and said second non-defibrillation lead, and to determine a condition of at least one of said defibrillation lead and said non-defibrillation lead according to the measured impedance value.

A twenty-fifth aspect of the present disclosure, as a variant of the twenty-fourth aspect, relates to a device, wherein said second non-defibrillation lead is configured for applying cardiac contractility modulation stimulation.

A twenty-sixth aspect of the present disclosure, as a variant of the twenty-fifth aspect, relates to a device, wherein said circuitry is configured to time said test pulse during applying of the cardiac contractility modulation stimulation via said second non-defibrillation lead.

A twenty-seventh aspect of the present disclosure, as a variant of the twenty-fifth aspect, relates to a device, wherein said circuitry is configured to time said test pulse during a ventricle refractory period.

A twenty-eighth aspect of the present disclosure, as a variant of any one of the twenty-fourth to twenty-seventh aspect, relates to a device, wherein said defibrillation lead comprises a coil, and said non-defibrillation lead comprises a ring electrode and a tip electrode; and wherein said impedance is measured between at least one of: said coil and said ring electrode; said coil and said tip electrode.

A twenty-ninth aspect of the present disclosure, as a variant of any one of the twenty-fourth to twenty-eighth aspect, relates to a device, wherein said circuitry is configured to determine said condition of at least one of said defibrillation lead and said non-defibrillation lead by one or more of: comparing said measured impedance to one or more of: a look-up table; one or more previously measured impedance values; one or more predefined impedance values.

A thirtieth aspect of the present disclosure, as a variant of any one of the twenty-fourth to twenty-ninth aspect, relates to a device, wherein said circuitry is configured to time said test pulse to perform measurement once every 24 hours.

A thirty-first aspect of the present disclosure, as a variant of any one of the twenty-fourth to thirtieth aspect, relates to a device, wherein said circuitry is configured to generate and issue an alert regarding said determined lead condition.

A thirty-second aspect of the present disclosure, as a variant of any one of the twenty-fourth to thirty-first aspect, relates to a device, wherein said determined lead condition comprises at least one of: lead fracture, dislodgment of the lead, lead insulation, lead connectivity, lead deformation.

A thirty-third aspect of the present disclosure, as a variant of any one of the twenty-fourth to thirty-second aspect, relates to a device, wherein said defibrillation lead comprises a coil, ring electrode and tip electrode; and said non-defibrillation lead comprises a ring electrode and a tip electrode; and said impedance is measured between one of said coil, ring electrode and tip electrode of said defibrillation lead and one of said ring electrode and tip electrode of said non-defibrillation lead.

A thirty-fourth aspect of the present disclosure, as a variant of any one of the twenty-fourth to thirty-third aspect, relates to a device, wherein said non-defibrillation lead comprises a cardiac contractility modulation (cardiac contractility modulation) lead or a pacing lead.

A thirty-fifth aspect of the present disclosure relates to an implantable cardiac device comprising: a housing; a defibrillation lead having a coil, said defibrillation lead extending from said housing; a non-defibrillation lead having at least one electrode, said non-defibrillation lead extending from said housing; circuitry for controlling and activating said defibrillation lead and said non-defibrillation lead and for measuring impedance, said circuitry configured to: apply a test pulse to measure a baseline impedance between said coil of said defibrillation lead and said housing; apply a test pulse to measure a baseline impedance between said at least one electrode of said non-defibrillation lead and said coil of said defibrillation lead; apply, periodically, a test pulse to measure impedance between said at least one electrode of said non-defibrillation lead and said coil of said defibrillation lead; and estimate, according to a difference between a currently measured impedance level measured between said at least one electrode of said non-defibrillation lead and said coil of said defibrillation lead and one or both of said baseline impedance measurements, a current impedance between said coil of said defibrillation lead and said housing; and assess a condition of said defibrillation lead based on said estimated current impedance.

A thirty-sixth aspect of the present disclosure, as a variant of the thirty-fifth aspect, relates to a device, wherein said circuitry is configured to apply said test pulse to measure impedance between said at least one electrode of said non-defibrillation lead and said coil of said defibrillation lead at least every 24 hours.

The present invention is exclusively defined in the following claims.

**Claims**

1. An implantable cardiac device comprising:

   a first defibrillation lead including a coil, a ring electrode and a tip electrode;
   a second non-defibrillation lead including a ring electrode and a tip electrode; and
   circuitry for controlling and activating said coil and electrodes, said circuitry including at least one grounded resistor electrically connected to said second non-defibrillation lead; said circuitry configured to measure current across said grounded resistor in response to an applied test pulse to obtain an indication of a condition of at least said first defibrillation lead or a portion thereof.

2. The device according to claim 1, wherein said circuitry is at least partially disposed within a housing of said cardiac device, said leads extending from within said housing.

3. The device according to any of claims 1-2, wherein said current measured across said grounded resistor in response to an applied test pulse is used for calculating impedance between said coil and said device housing.

4. The device according to claim 3, wherein said circuitry is configured to calculate said impedance between said coil and a housing of said device using a pre-determined linearizing factor.

5. The device according to any of claims 1-4, wherein said circuitry is programmed for timing said test pulse during an expected total refractory period of the cardiac cycle.

6. The device according to any of claims 1-5, wherein said non-defibrillation lead is a cardiac contractility modulation lead, and said circuitry is programmed for timing said measurement of current on said grounded resistor during applying of a cardiac contractility modulation signal.

FIG. 1A

FIG. 1B

FIG. 1C

201 — Using existing device circuitry, measure impedance between a defibrillation lead and a non-defibrillation lead by applying a test pulse

203 — Compare the measured impedance value to a look up table, to previously measured values, and/or to predefined values

204 — Determine a condition of the lead

205 — Optionally repeat

FIG. 2A

209 — In a device comprising a defibrillation lead and at least one cardiac contractility modulation lead, measure impedance during applying of cardiac contractility modulation stimulation

211 — Compare the measured impedance value to a look up table, to previously measured values, and/or to predefined values

213 — Determine a condition of the lead

215 — Optionally repeat

FIG. 2B

FIG. 2C

FIG. 2D

FIG. 3

FIG. 4

FIG. 5

FIG. 6

| Impedance value range | Status |
| --- | --- |
| < 50 Ohms | Short circuit |
| 50 - 150 Ohms | Lead isolation problem |
| 150-1000 Ohms | Normal range |
| > 1000 Ohms | Conductor failure |

FIG. 7

FIG. 8

FIG.9

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 62957245

**Non-patent literature cited in the description**

- **CHARLES D. SWERDLOW;** ; **PAUL A. FRIEDMAN**. Advanced ICD Troubleshooting: Part II. *Pacing Clin Electrophysiol*, 2006, vol. 29 (1), 70-96